# EUROPEAN PATENT SPECIFICATION

(11) **EP 1 531 157 B1**
(45) Date of publication and mention of the grant of the patent: **19.03.2008**
(21) Application number: 05075063.7
(22) Date of filing: 28.02.2001
(51) Int. Cl.: C07F 9/58, C07F 9/6561

(54) **Cardioprotective phosphonates**
Cardioprotektive Phosphonate
Phosphonates cardioprotecteurs

(30) Priority: 29.02.2000 US 185899 P
(43) Date of publication of application: 18.05.2005
(62) Divisional of application: 01909391.3
(73) Proprietor: Medicure International Inc., Holetown Saint James (BB)
(72) Inventor: Haque, Wasimul, Edmonton, Alberta, T6L 1E7 (CA)
(74) Representative: Evens, Paul Jonathan

(56) References cited:
- WO-A-98/28310
- WO-A-99/53928
- CA-A1- 2 254 528
- PATENT ABSTRACTS OF JAPAN vol. 1998, no. 11, 30 September 1998 (1998-09-30) -& JP 10 158244 A (KISSEI PHARMACEUT CO LTD), 16 June 1998 (1998-06-16)
- ARBUZOV, S. YA.: "Synthesis and pharmacological investigation of some new compounds related structurally to some natural metabolites" CONF. HUNG. THER. INVEST. PHARMACOL., SOC. PHARMACOL. HUNG., 4TH , MEETING DATE 1966, 489-502. EDITOR(S): DUMBOVICH, B. PUBLISHER: AKAD. KIADO, BUDAPEST, HUNG. CODEN: 21PFAR, 1968, XP001205096
- DATABASE CA [Online] CHEMICAL ABSTRACTS SERVICE, COLUMBUS, OHIO, US; ARBUZOV, S. YA.: "Pharmacological properties of the products of the condensation of phenamine with some metabolites" XP002170150 retrieved from STN Database accession no. 1968:458274 -& FARMAKOLOGIYA I TOKSIKOLOGIYA, vol. 31, no. 3, 1968, pages 373-376, XP001027516 ISSN: 0014-8318

## Description

### FIELD OF THE INVENTION

This invention relates to pyridoxine phosphonate analogues, to pharmaceutical compositions thereof, and to treatments for cardiovascular and related diseases, for example, hypertrophy, hypertension, congestive heart failure, myocardial ischemia, arrhythmia, heart failure subsequent to myocardial infarction, myocardial infarction, ischemia reperfusion injury, and diseases that arise from thrombotic and prothrombotic states in which the coagulation cascade is activated; and treatments for diabetes mellitus and related diseases, for example, hyperinsulinemia, diabetes-induced hypertension, obesity, insulin resistance, and damage to blood vessels, eyes, kidneys, nerves, autonomic nervous system, skin connective tissue, or immune system.

### BACKGROUND

Pyridoxal-5'-phosphate (PLP), an end product of vitamin B₆ metabolism, plays a vital role in mammalian health. In previous patents (US 6,051,587 and US 6,043,259) the role of pyridoxal-5'-phosphate, and its precursors pyridoxal and pyridoxine (vitamin B₆), in mediating cardiovascular health and in treating cardiovascular related diseases is disclosed.

The major degradation pathway for pyridoxal-5'-phosphate *in vivo* is the conversion to pyridoxal, catalysed by alkaline phosphatase. Thus, there is a need to identify and administer drugs that are functionally similar to pyridoxal-5'-phosphate such as pyridoxine phosphonate analogues or pyridoxine malonate analogues, that elicit similar or enhanced cardiovascular benefits, and that beneficially affect PLP-related conditions, but are stable to degradation by phosphatase.

International application WO 98/28310 A reports that aminophosphonic acid derivatives have lipoprotein lowering activity.

The abstract of Japanese Patent JP 10 158544A reports the synthesis of a pyridoxamine derivative having a Maillard reaction inhibiting activity.

Arbuzoy, S.J., "Synthesis and pharmacological investigation of some new compounds related structurally to some natural metabolites", Conf. Hung, Ther. Invest. Pharmacol. Soc. Pharmacol. Hung., 4th (1996), pages 489-502, reports the synthesis of new alkyl derivatives of phenamine, some of them demonstrating a hypotensive effect.

The abstract of Arbuzov, S,YA, "Pharmocological properties of the products of the condensation of phenamine with metabolities", Farmakol. Toksikol (1968). Vol 31. No. 3, pages 373-6, reports that Incorporation Into a phenamine molecule of benzoic acid, pyridoxine, alanine and other metabolites can produce compounds whose sympathomimetic properties are either attenuated or wholly absent.

### SUMMARY OF THE INVENTION

The present invention provides for pyridoxine phosphonate analogues.

In one aspect, the present invention includes a compound of formula II: 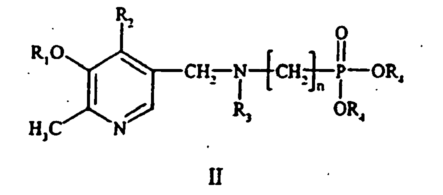 in which
R₁ is hydrogen or alkyl;
R₂ is -CHO, -CH₂OH, -CH₃ or -CO₂R₅ in which R₅ is hydrogen, alkyl, or aryl; or
R₂ is -CH₂O-alkyl- in which alkyl is covalently bonded to the oxygen at the 3-position instead of R₁;
R₃ is hydrogen, alkyl, aryl, or aralkyl;
R₄ is hydrogen, alkyl, aryl, aralkyl, or -CO₂R₆ in which R₆ is hydrogen, alkyl, aryl, or aralkyl; and
n is 1 to 6;
or a pharmaceutically acceptable acid addition salt thereof.

In another aspect, the invention is directed to pharmaceutical compositions that include a pharmaceutically acceptable carrier and a therapeutically effective amount of at least one compound of formula II.

In another aspect, invention is directed to a use of a compound of formula II for the preparation of a medicament for treating cardiovascular and related diseases, for example, hypertension, hypertrophy, arrhythmia, congestive heart failure, myocardial ischemia, heart failure subsequent to myocardial infarction, myocardial infarction, ischemia reperfusion injury, and diseases that arise from thrombotic and prothrombotic states in which the coagulation cascade is activated, the medicament comprising a therapeutically effective amount of at least one compound of formula II in a unit dosage form. For such a treatment, the compound of formula II may be for administration alone or concurrently with a known therapeutic cardiovascular agent, for example, angiotensin converting enzyme inhibitor, an angiotensin II receptor antagonist, a vasodilator, a diuretic, an α-adrenergic receptor antagonist, a β-adrenergic receptor antagonist, an antioxidant, or a mixture thereof.

In still another aspect, the invention is directed to a use of a compound of formula II for the preparation of a medicament for treating diabetes mellitus and related diseases, for example, hyperinsulinemia, insulin resistance, obesity, diabetes-induced hypertension, and damage to eyes, kidneys, blood vessels, nerves, autonomic nervous system, skin, connective tissue, or immune system, the medicament comprising a therapeutically effective amount of a compound of formula II in a unit dosage form. For such a treatment, the compound of formula II may be for administration alone or concurrently with known medicaments suitable for treating diabetes mellitus and related diseases, for example, insulin, hypoglycemic drugs, or a mixture thereof

### DESCRIPTION OF THE INVENTION

The present invention provides for pyridoxine phosphonate analogues such as, for example, (2-methyl-3-hydroxy-4-hydroxymethyl-5-pyridyl)azaalkylphosphonates) pharmaceutical compositions, and medicaments for treatment of cardiovascular and related diseases, and diabetes mellitus and related diseases.

It is to be understood that the recitation of numerical ranges by endpoints includes all numbers and fractions subsumed within that range (e.g. 1 to 5 includes 1, 1.5, 2, 2.75, 3, 3.80, 4, and 5).

It is to be understood that all numbers and fractions thereof are presumed to be modified by the term "about."

It is to be understood that "a," "an," and "the" include plural referents unless the content clearly dictates otherwise. Thus, for example, reference to a composition containing "a compound" includes a mixture of two or more compounds.

It is to be understood that some of the compounds described herein contain one or more asymmetric centers and may thus give rise to enantiomers, diasteriomers, and other stereoisomeric forms which may be defined in terms of absolute stereochemistry as (R)- or (S)-. The present invention is meant to imclude all such possible diasteriomers and enantiomers as well as their racemic and optically pure forms. Optically active (R)- and (S)- isomers may be prepared using chiral synthons or chiral reagents, or resolved using conventional techniques. When the compounds described herein contain olefinic double bonds or other centers of geometric asymmetry, and unless specified otherwise, it is intended that the compounds include both E and A geometric isomers. Likewise all tautomeric forms are intended to be included.

The general definitions used herein have the following meanings within the scope of the present invention.

As used herein the term "alkyl" includes a straight or branched saturated aliphatic hydrocarbon redicals, such as, for example, methyl, ethyl, propyl, isopropyl (1-methylethyl), 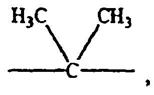 butyl, *tert*-butyl (1,1-dimethylethyl), and the like.

As used herein the term "alkoxy" refers to -O-alkyl with alkyl as defined above. Alkoxy groups include those with 1 to 4 carbon atoms in a straight or branched chain, such as, for example, methoxy, ethoxy, propoxy, isopropoxy (1-methylethoxy), butoxy, *tert*-butoxy (1,1-dimethylethoxy), and the like.

As used herein the term "aryl" refers to unsaturated aromatic carbocyclic radicals having a single ring, such as phenyl, or multiple condensed rings, such as naphthyl or anthryl. The term "aryl" also includes substituted aryl comprising aryl substituted on a ring by, for example, C₁₋₄ alkyl, C₁₋₄ alkoxy, amino, hydroxy, phenyl, nitro, halo, carboxyalkyl or alkanoyloxy. Aryl groups include, for example phenyl, naphthyl, anthryl, biphenyl, methoxyphenyl, halophenyl, and the like.

As used herein the term "alkylamino" refers to -N-alkyl with alkyl as defined above. Alkylamino groups include those with 1-6 carbons in a straight or branched chain, such as, for example, methylamino, ethylamino, propylamino, and the like.

As used herein the term "arylamino" refers to -N-aryl with aryl as defined above. Arylamino includes -NH-phenyl, -NH-biphenyl, -NH-4-methoxyphenyl, and the like.

As used herein the term "aralkyl" refers to an aryl radical defined as above substituted with an alkyl radical as defined above (e.g. aryl-alkyl-). Aralkyl groups include, for example, phenethyl, benzyl, and naphthylmethyl.

As used herein the term "halo" includes bromo, chloro, and fluoro. Preferably halo is fluoro.

As used herein the term "alkylcarbonyloxy" includes alkyl as defined above bonded to carbonyl bonded to oxygen, such as, for example, acetate, propionate and t-butylcarbonyloxy

Cardiovascular and related diseases include, for example, hypertension, hypertrophy, congestive heart failure, heart failure subsequent to myocardial infarction, arrhythmia, myocardial ischemia, myocardial infarction, ischemia reperfusion injury, and diseases that arise from thrombotic and prothrombotic states in which the coagulation cascade is activated.

Heart failure is a pathophysiological condition in which the heart is unable to pump blood at a rate commensurate with the requirement of the metabolizing tissues or can do so only from an elevated filling pressure (increased load). Thus, the heart has a diminished ability to keep up with its workload. Over time, this condition leads to excess fluid accumulation, such as peripheral edema, and is referred to as congestive heart failure.

When an excessive pressure or volume load is imposed on a ventricle, myocardial hypertrophy (i.e., enlargement of the heart muscle) develops as a compensatory mechanism. Hypertrophy permits the ventricle to sustain an increased load because the heart muscle can contract with greater force. However, a ventricle subjected to an abnormally elevated load for a prolonged period eventually fails to sustain an increased load despite the presence of ventricular hypertrophy, and pump failure can ultimately occur.

Heart failure can arise from any disease that affects the heart and interferes with circulation. For example, a disease that increases the heart muscle's workload, such as hypertension, will eventually weaken the force of the heart's contraction. Hypertension is a condition in which there is an increase in resistance to blood flow through the vascular system. This resistance leads to increases in systolic and/or diastolic blood pressures. Hypertension places increased tension on the left ventricular myocardium, causing it to stiffen and hypertrophy, and accelerates the development of atherosclerosis in the coronary arteries. The combination of increased demand and lessened supply increases the likelihood of myocardial ischemia leading to myocardial infarction, sudden death, arrhythmias, and congestive heart failure.

Ischemia is a condition in which an organ or a part of the body fails to receive a sufficient blood supply. When an organ is deprived of a blood supply, it is said to be hypoxic. An organ will become hypoxic even when the blood supply temporarily ceases, such as during a surgical procedure or during temporary artery blockage. Ischemia initially leads to a decrease in or loss of contractile activity. When the organ affected is the heart, this condition is known as myocardial ischemia, and myocardial ischemia initially leads to abnormal electrical activity. This can generate an arrhythmia. When myocardial ischemia is of sufficient severity and duration, cell injury can progress to cell death-i.e., myocardial infarction-and subsequently to heart failure, hypertrophy, or congestive heart failure.

When blood flow resumes to an organ after temporary cessation, this is known as ischemic reperfusion of the organ. For example, reperfusion of an ischemic myocardium can counter the effects of coronary occlusion, a condition that leads to myocardial ischemia. Ischemic reperfusion to the myocardium can lead to reperfusion arrhythmia or reperfusion injury. The severity of reperfusion injury is affected by numerous factors, such as, for example, duration of ischemia, severity of ischemia, and speed of reperfusion. Conditions observed with ischemia reperfusion injury include neutrophil infiltration, necrosis, and apoptosis.

Drug therapies, using known active ingredients such as vasodilators, angiotensin II receptor antagonists, angiotensin converting enzyme inhibitors, diuretics, antithrombolytic agents, α or β-adrenergic receptor antagonists, α-adrenergic receptor antagonists, calcium channel blockers, and the like, are available for treating cardiovascular and related diseases.

Diabetes mellitus and related diseases include hyperinsulinemia, insulin resistance, obesity, diabetes-induced hypertension, and damage to blood vessels, eyes, kidneys, nerves, autonomic nervous system, skin, connective tissue, and immune system. Diabetes mellitus is a condition in which blood glucose levels are abnormally high because the body is unable to produce enough insulin to maintain normal blood glucose levels or is unable to adequately respond to the insulin produced. Insulin-dependent diabetes mellitus (often referred to as type I diabetes) arises when the body produces little or no insulin. About 10% of all diabetics have type I diabetes. Noninsulin-dependent diabetes mellitus (often referred to as type II diabetes) arises when the body cannot adequately respond to the insulin that is produced in response to blood glucose levels.

Available treatments include weight control, exercise, diet, and drug therapy. Drug therapy for type I diabetes mellitus requires the administration of insulin; however, drug therapy for type II diabetes mellitus usually involves the administration of insulin and/or oral hypoglycemic drugs to lower blood glucose levels. If the oral hypoglycemic drugs fail to control blood sugar, then insulin, either alone or concurrently with the hypoglycemic drugs, will usually be administered.

The invention is generally directed to pyridoxine phosphonate analogues such as, for example, (2-methyl-3-hydroxy-4-hydroxymethyl-5-pyridyl)azaalkylphosphonates) compositions including these analogues, and uses of such analogues for the preparation of a medicament containing a therapeutically effective amount of at least one of these analogues to treat cardiovascular and related diseases or diabetes and related diseases.

To enhance absorption from the digestive tract and across biological membranes, polar groups on a drug molecule can be blocked with lipophilic functions that will be enzymatically cleaved off from the drug after absorption into the circulatory system. Lipophilic moieties can also improve site-specificity and biovailability of the drug. The speed at which the blocking groups are removed can be used to control the rate at which the drug is released. The blocking of polar groups on the drug can also slow first-pass metabolism and excretion. An ester is a common blocking group that is readily hydrolyzed from the drug by endogenous esterases. Bundgaard, Design and Application of Prodrugs in A Textbook of Drug Design and Development (Krogsgaard-Larson & Bundgaard, eds., Hardwood Academic Publishers, Reading, United Kingdom 1991).

In one embodiment, the compounds of the present invention are analogues of pyridoxal phosphonate.

The compounds of the invention include (2-methyl-3-hydroxy-4-hydroxymethyl-5-pyridyl)azaalkylphosphonate analogues. Such compounds are represented by formula II: 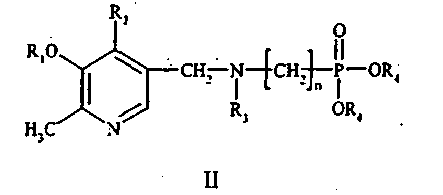 in which
R₁ is hydrogen or alkyl;
R₂ is -CHO, -CH₂OH, -CH₃ or -CO₂R₅ in which R₃ is hydrogen, alkyl, or aryl; or
R₂ is -CH₂O-alkyl- in which alkyl is covalently bonded to the oxygen at the 3-position instead of R₁;
R₃ is hydrogen, alkyl, aryl, or aralkyl;
R₄ is hydrogen, alkyl, aryl, aralkyl, or -CO₂R₆ in which R₆ is hydrogen, alkyl, aryl, or aralkyl; and
n is 1 to 6;
or a pharmaceutically acceptable acid addition salt thereof.

Examples of compounds of formula II include those where R₁ is hydrogen, or those where R₂ is -CH₂OH, or -CH₂-O-alkyl- in which alkyl is covalently bonded to the oxygen at the 3-position instead of R₁, or those where R₃ is hydrogen, or those where R₁ is alkyl or hydrogen. Additional examples of compounds of formula II include those where R₁ is ethyl.

Pharmaceutically acceptable acid addition salts of the compounds of formula II include salts derived from nontoxic inorganic acids such as hydrochloric, nitric, phosphoric, sulfuric, hydrobromic, hydriodic, hydrofluoric, phosphorus, and the like, as well as the salts derived from nontoxic organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate; bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate, n-methyl glutamine, etc. (see, e.g., Berge et al., J.Pharmaceutical Science. 66: 1-19 (1977)).

The acid addition salts of the basic compounds are prepared by contacting the free base form with a sufficient amount of the desired acid to produce the salt in the conventional manner. The free base form can be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

### Syntheses

To prepare a compound of formula II, 3,4-isopropylidenepy ridoxine-5-amine is used as a starting material. The amine is treated with a haloalkylphosphonate diester, such as diethyl bromomethylphosphonate, to give 5'-phosphonoazaalkylpyridine diesters. Reaction of the 3,4-isopropylidene-5'-phosphonoazaalkylpyridoxine diesters with a trialkylsilyl halide, such as trimethylsilyl bromide, in an aprotic solvent, such as acetonitrile, removes the ester groups of the phosphonate diester to provide the corresponding free 3,4-isopropylidene-5'-phosphonoazaalkylpyridoxine diacid. The acetonide protecting group on the 3 and 4 position of the pyridoxine ring on the 3,4-isopropylidene-5'-phosphonoazaalkylpyridoxine diacid can be removed by reaction with acid and water, such as 20% water in acetic acid as can be seen in the following scheme.

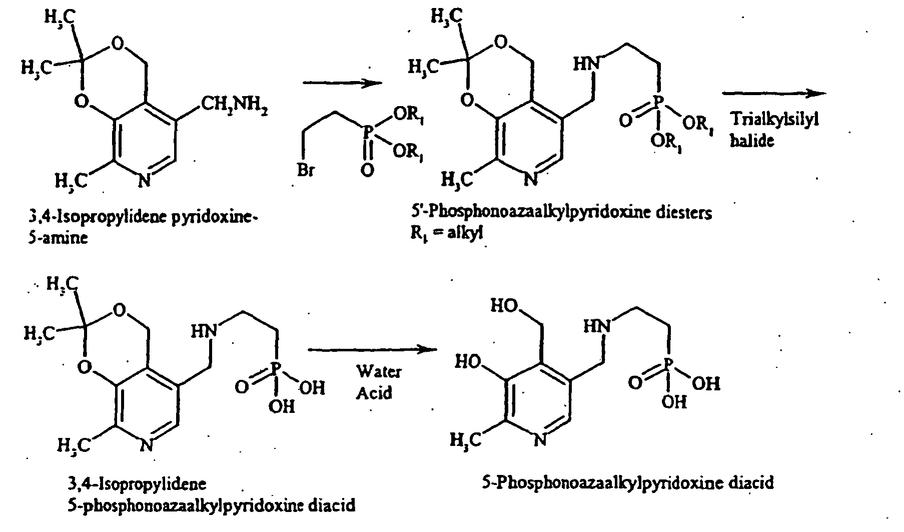

One skilled in the art would recognize variations in the sequence of steps and would recognize variations in the appropriate reaction conditions from the analogous reactions shown or otherwise known that can be appropriately used in the above-described processes to make the compounds of formula II herein.

The products of the reactions described herein are isolated by conventional means such as extraction, distillation, chromatography, and the like.

### Methods of Use

In accordance with the present invention, the analogues can be used in the treatment of cardiovascular and related diseases; and in the treatment of diabetes mellitus and related diseases.

Cardiovascular and related diseases include, for example, hypertension, hypertrophy, congestive heart failure, heart failure subsequent to myocardial infarction, arrhythmia, myocardial ischemia, myocardial infarction, ischemia reperfusion injury, and diseases that arise from thrombotic and prothrombotic states in which the coagulation cascade is activated.

Diabetes mellitus and related diseases include, for example, hyperinsulinemia, insulin resistance, obesity, diabetes-induced hypertension, and damage to blood vessels, eyes, kidneys, nerves, autonomic nervous system, skin, connective tissue, and immune system.

"Treatment" and "treating" as used herein include preventing, inhibiting, alleviating, and healing cardiovascular and related diseases; diabetes mellitus and related diseases; or symptoms thereof. Treatment can be carried out by administering a therapeutically effective amount of a compound of the invention. A "therapeutically effective amount" as used herein includes a prophylactic amount, for example, an amount effective for preventing or protecting against cardiovascular and related diseases; diabetes mellitus and related diseases; or symptoms thereof, and amounts effective for alleviating or healing cardiovascular and related diseases; or diabetes mellitus and related diseases; or symptoms thereof.

A physician or veterinarian of ordinary skill readily determines a subject who is exhibiting symptoms of any one or more of the diseases described above. Regardless of the route of administration selected, the compounds of the present invention of formula II or a pharmaceutically acceptable acid addition salt thereof can be formulated into pharmaceutically acceptable unit dosage forms by conventional methods known to the pharmaceutical art. An effective but nontoxic quantity of the compound is employed in treatment. The compounds can be administered in enteral unit dosage forms, such as, for example, tablets, sustained-release tablets; enteric coated tablets, capsules, sustained-release capsules, enteric coated capsules, pills, powders, granules, solutions, and the like. They can also be administered parenterally, such as, for example, subcutaneously, intramuscularly, intradermally, intramammarally, intravenously, and other administrative methods known in the art.

Although it is possible for a compound of the invention to be administered alone in a unit dosage form, preferably the compound is administered in admixture as a pharmaceutical composition. A pharmaceutical composition comprises a pharmaceutically acceptable carrier and at least one compound of formula II or a pharmaceutically acceptable acid addition salt thereof. A pharmaceutically acceptable carrier includes, but is not limited to, physiological saline, ringers, phosphate-buffered saline, and other carriers known in the art. Pharmaceutical compositions can also include additives, for example, stabilizers, antioxidants, colorants, excipients, binders, thickeners, dispersing agents, readsorpotion enhancers, buffers, surfactants, preservatives, emulsifiers, isotonizing agents, and diluents. Pharmaceutically acceptable carriers and additives are chosen such that side effects from the pharmaceutical compound are minimized and the performance of the compound is not canceled or inhibited to such an extent that treatment is ineffective.

Methods of preparing pharmaceutical compositions containing a pharmaceutically acceptable carrier and at least one compound of formula II or a pharmaceutically acceptable acid addition salt thereof are known to those of skill in the art. All methods can include the step of bringing the compound of the invention in association with the carrier and additives. The formulations generally are prepared by uniformly and intimately bringing the compound of the invention into association with a liquid carrier or a finely divided solid carrier or both, and then, if necessary, shaping the product into the desired unit dosage form.

The ordinarily skilled physician or veterinarian will readily determine and prescribe the therapeutically effective amount of the compound to treat the disease for which treatment is administered. In so proceeding, the physician or veterinarian could employ relatively low dosages at first, subsequently increasing the dose until a maximum response is obtained. Typically, the particular disease, the severity of the disease, the compound to be administered, the route of administration, and the characteristics of the mammal to be treated, for example, age, sex, and weight, are considered in determining the effective amount to administer. Administering a therapeutic amount of a compound of the invention for treating cardiovascular and related diseases; diabetes mellitus and related diseases; or symptoms thereof, is in a range of 0.1-100 mg/kg of a patient's body weight, more preferably in the range of 0.5-50 mg/kg of a patient's body weight, per daily dose. The compound can be administered for periods of short and long duration. Although some individual situations can warrant to the contrary, short-term administration, for example, 30 days or less, of doses larger than 25 mg/kg of a patient's body weight is preferred to long-term administration. When long-term administration, for example, months or years, is required, the suggested dose should not exceed 25 mg/kg of a patient's body weight.

A therapeutically effective amount of a compound for treating the above-identified diseases or symptoms thereof can be administered prior to, concurrently with, or after the onset of the disease or symptom.

The compound also can be administered to treat cardiovascular and related diseases, for example, hypertrophy, hypertension, congestive heart failure, heart failure subsequent to myocardial infarction, myocardial ischemia, ischemia reperfusion injury, arrhythmia, or myocardial infarction. Preferably, the cardiovascular disease treated is hypertrophy or congestive heart failure. Still preferably, the cardiovascular disease treated is arrhythmia. Also preferably, the cardiovascular disease treated is ischemia reperfusion injury.

The compound can also be administered to treat cardiovascular diseases and other diseases that arise from thrombotic and prothrombotic states in which the coagulation cascade is activated, such as, for example, deep vein thrombosis, disseminated intravascular coagulopathy, Kasabach-Merritt syndrome, pulmonary embolism, myocardial infarction, stroke, thromboembolic complications of surgery, and peripheral arterial occlusion. A compound of the invention may also be useful in the treatment of adult respiratory distress syndrome, septic shock, septicemia, or inflammatory responses, such as edema and acute or chronic atherosclerosis, because thrombin has been shown to activate a large number of cells outside of the coagulation process, such as, for example, neutrophils, fibroblasts, endothelial cells, and smooth muscle cells.

Moreover, the compound can be administered concurrently with compounds that are already known to be suitable for treating the above-identified diseases. For example, treatments include concurrently administering at least one compound of formula II a pharmaceutically acceptable acid addition salt thereof, or a mixture thereof with a therapeutic cardiovascular compound to treat hypertrophy, hypertension, congestive heart failure, heart failure subsequent to myocardial infarction, myocardial ischemia, ischemia reperfusion injury, arrhythmia, or myocardial infarction. Preferably the cardiovascular disease treated is hypertrophy or congestive heart failure. Still preferably, the cardiovascular disease treated is arrhythmia. Also preferably; the cardiovascular disease treated is ischemia reperfusion injury.

Therapeutic cardiovascular compounds that can be concurrently administered with at least one compound of the invention include an angiotensin converting enzyme inhibitor, an angiotensin II receptor antagonist, a calcium channel blocker, an antithrombolytic agent, a β-adrenergic receptor antagonist, a vasodilator, a diuretic, an α-adrenergic receptor antagonist, an antioxidant, and a mixture thereof. A compound of the invention also can be concurrently administered with PPADS (pyridoxal phosphate-6-azophenyl-2',4'-disulphonic acid), also a therapeutic cardiovascular compound, or with PPADS and another known therapeutic cardiovascular compound as already described.

Preferably a therapeutic cardiovascular compound, which is concurrently administered with at least one compound of formula II, a pharmaceutically acceptable acid addition salt thereof, or a mixture thereof, is an angiotensin converting enzyme inhibitor, an angiotensin II receptor antagonist, or a diuretic. Still preferably, the therapeutic cardiovascular compound is an α-adrenergic receptor antagonist. Also preferably, the therapeutic cardiovascular compound is a calcium channel blocker.

These therapeutic cardiovascular compounds are generally used to treat cardiovascular and related diseases as well as symptoms thereof. A skilled physician or veterinarian readily determines a subject who is exhibiting symptoms of any one or more of the diseases described above and makes the determination about which compound is generally suitable for treating specific cardiovascular conditions and symptoms.

For example, myocardial ischemia can be treated by the administration of, for example, angiotensin converting enzyme inhibitor, an angiotensin II receptor antagonist, a calcium channel blocker, an anti thrombolytic agent, a β-adrenergic receptor antagonist, a diuretic, an α-adrenergic receptor antagonist, or a mixture thereof. In some instances, congestive heart failure can be treated by the administration of, for example, angiotensin converting enzyme inhibitor, an angiotensin II receptor antagonist, a calcium channel blocker, a vasodilator, a diuretic, or a mixture thereof.

Myocardial infarction can be treated by the administration of, for example, angiotensin converting enzyme inhibitor, a calcium channel blocker, an antithrombolytic agent, a β-adrenergic receptor antagonist, a diuretic, an α-adrenergic receptor antagonist, or a mixture thereof.

Hypertension can be treated by the administration of, for example, angiotensin converting enzyme inhibitor, a calcium channel blocker, a β-adrenergic receptor antagonist, a vasodilator, a diuretic, an α-adrenergic receptor antagonist, or a mixture thereof.

Moreover, arrhythmia can be treated by the administration of for example, a calcium channel blocker, a β-adrenergic receptor antagonist, or a mixture thereof.

Antithrombolytic agents are used for reducing or removing blood clots from arteries.

Hypertrophy can be treated by the administration of, for example, an angiotensin converting enzyme inhibitor, an angiotensin II receptor antagonist, a calcium channel blocker, or a mixture thereof.

Ischemia reperfusion injury can be treated by the administration of, for example, an angiotensin converting enzyme inhibitor, an angiotensin II receptor antagonist, a calcium channel blocker, or a mixture thereof.

Known angiotensin converting enzyme inhibitors include, for example, captopril, enalapril, lisinopril, benazapril, fosinopril, quinapril, ramipril, spirapril, imidapril, and moexipril.

Examples of known angiotensin II receptor antagonists include both angiotensin I receptor subtype antagonists and angiotensin II receptor subtype antagonists. Suitable antiotensin II receptor antagonists include losartan and valsartan.

Suitable calcium channel blockers include, for example, verapamil, diltiazem, nicardipine, nifedipine, amlodipine, felodipine, nimodipine, and bepridil.

Antithrombolytic agents known in the art include antiplatelet agents, aspirin, and heparin.

Examples of known β-adrenergic receptor antagonists include atenolol, propranolol, timolol, and metoprolol.

Suitable vasodilators include, for example, hydralazine; nitroglycerin, and isosorbide dinitrate.

Suitable diuretics include, for example, furosemide, diuril, amiloride, and hydrodiuril.

Suitable α-adrenergic receptor antagonists include, for example, prazosin, doxazocin, and labetalol.

Suitable antioxidants include vitamin E, vitamin C, and isoflavones.

At least one compound of formula II, a pharmaceutically acceptable acid addition salt thereof, or a mixture thereof and a therapeutic cardiovascular compound can be administered concurrently. "Concurrent administration" and "concurrently administering" as used herein includes administering a compound of the invention and a therapeutic cardiovascular compound in admixture, such as, for example, in a pharmaceutical composition or in solution, or as separate compounds, such as, for example, separate pharmaceutical compositions or solutions administered consecutively, simultaneously, or at different times but not so distant in time such that the compound of the invention and the therapeutic cardiovascular compound cannot interact and a lower dosage amount of the active ingredient cannot be administered.

At least one compound of formula II, a pharmaceutically acceptable acid addition salt thereof, or a mixture thereof also can be administered to treat diabetes mellitus and related diseases. Preferably the disease treated is type I diabetes, type II diabetes, or obesity. Also preferably, the disease treated is damage to blood vessels, eyes, kidneys, nerves, autonomic nervous system, skin, connective tissue, or immune system. Still preferably, the disease treated is insulin resistance or hyperinsulinemia. And preferably, the disease treated is diabetes-induced hypertension.

The use of the invention also includes concurrent administration of at least one compound of formula II, a pharmaceutically acceptable acid addition salt thereof, or a mixture thereof with insulin and/or a hypoglycemic compound to treat diabetes mellitus and related diseases. The compound can be administered concurrently with insulin and/or a hypoglycemic compound to treat type I diabetes, type II diabetes, or obesity. Preferably the compound can be administered concurrently with insulin and/or hypoglycemic compound to treat damage to blood vessels, eyes, kidneys, nerves, autonomic nervous system, skin, connective tissue, or immune system. Still preferably, the compound can be administered concurrently with insulin and/or hypoglycemic compound to treat insulin resistance or hyperinsulinemia. Also preferably, the compound can be administered concurrently with insulin and/or hypoglycemic compound to treat diabetes-induced hypertension.

A compound typically can be administered concurrently with insulin to treat type I diabetes, type II diabetes, and related conditions and symptoms. For type II diabetes, insulin resistance, hyperinsulinemia, diabetes-induced hypertension, obesity, or damage to blood vessels, eyes, kidneys, nerves, autonomic nervous system, skin, connective tissue, or immune system, a compound can be administered concurrently with a hypoglycemic compound instead of insulin. Alternatively, a compound can be administered concurrently with insulin and a hypoglycemic compound to treat type II diabetes, insulin resistance, hyperinsulinemia, diabetes-induced hypertension, obesity, or damage to blood vessels, eyes, kidneys, nerves, autonomic nervous system, skin, connective tissue, or immune system.

"Concurrent administration" and "concurrently administering" as used herein includes administering at least one compound of formula, II a pharmaceutically acceptable acid addition salt thereof, or a mixture thereof and insulin and/or a hypoglycemic compound in admixture, such as, for example, in a pharmaceutical composition, or as separate compounds, such as, for example, separate pharmaceutical compositions administered consecutively, simultaneously, or at different times. Preferably, if the compound and insulin and/or hypoglycemic compound are administered separately, they are not administered so distant in time from each other that the compound and the insulin and/or hypoglycemic compound cannot interact and a lower dosage amount of insulin and/or hypoglycemic compound cannot be administered.

Suitable hypoglycemic compounds include, for example, metformin, acarbose, acetohexamide, glimepiride, tolazamide, glipizide, glyburide, tolbutamide, chlorpropamide, and a mixture thereof. Preferably the hypoglycemic compound is tolbutamide.

This invention will be further characterized by the following examples. These examples are not meant to limit the scope of the invention, which has been fully set forth in the foregoing description. Variations within the scope of the invention will be apparent to those skilled in the art.

### EXAMPLES

All reagents used in the following Examples can be purchased from Aldrich Chemical Company (Milwaukee, WI or Allentown, PA).

### Example 1: Synthesis of di-t-butyl (α⁴,3-O-isopropylidene-3-hydroxy-4-hydroxymethyl-2-methyl-5-pyridyl)-3-azabutylphosphonate

(α⁴,3-O-Isopropylidene-3-hydroxy-4-hydroxymethyl-2-methyl-5-pyridyl)methylbromide (Imperalli et al, J. Org. Chem., 60, 1891-1894 (1995)) ( 1.08 g. 4.0 mmol) in anhydrous DMF (20 ml) was treated with sodium azide (260 mg, 4.0 mmol) at room temperature. After one hour stirring at room temperature, the solution was extracted with diethyl ether (5 x 20 mL). The combined extracts were washed with water (10 mL), and brine (10 mL) and dried (MgSO₄). The solvent was evaporated and the crude product was purified by chromatography on silica gel using ethyl ether: hexanes (2:1) as eluent to give the azide as a colorless liquid (552mg, 60%).
¹H NMR (CDC13, TMS) 1.57 (s, 6H), 2.42 (s, 3H), 4.23 (s, 2H), 4.86 (s, 2H), 7.96 (s, 1H).
The purified azide (100 mg, 0.4 mmol) was dissolved in 95% ethanol and hydrogenated at 1 atm in presence of Lindlar catalyst (50 mg) for one hour. The catalyst was removed by filtration (Celite), and the solvent removed to give the crude amine. Purification by chromatography on silica gel using CH₂Cl₂:MeOH (5:1) as eluent gave the product (80 mg, 82%) IHNMR (CD₂Cl₂) 1.53 (s, 6H), 2.34 (s, 3H), 3.72 (s, 2H), 4.91 (s, 2H), 5.31 (s, 2H), 7.93 (s, 1H).

The (α⁴,3-O-Isopropylidene-3-hydroxy-4-hydroxymethyl-2-methyl-5-pyridyl)methylamine, from above, (416 mg, 2 mmol) was heated in saturated, aqueous sodium bicarbonate solution (10 mL) to 95°C, followed by slow addition of diethyl 2-bromoethylphosphonate (0.09 mL, 0.5mmol) and the reaction stirred at 95° C overnight. The solution is evaporated using toluene to codistill the water. The crude product is triturated with ethyl acetate to dissolve the crude organic product. Chromatography on silica gel using methylene chloride:methanol:hexanes (5:1:5) gave 76 mg (41 %).
¹Hnmr (CDCl₃, TMS) 1.27 (t, 6H), 1.51 (s, 6H), 1.91 (t, 2H), 2.35 (s, 3H), 2.85 (t, 2H), 3.62 (s, 2H), 4.03 (m, 4H), 4.91 (s, 2H), 7.88 (s, 1H).
³¹P NMR (H-decoupled, CDCl₃): 31.00 (s)
This structure can be represented by formula XIX:

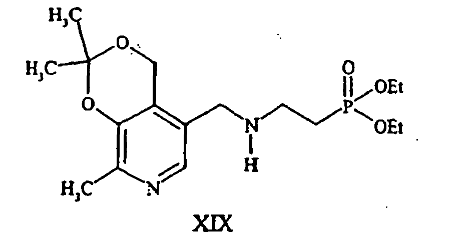

### Example 2: Synthesis of (α⁴,3-O-isopropylidene-3phydroxy-4-hydroxymethyl-2-methyl-5-pyridyl)-3-azabutylphosphonic acid

The product of Example 15, of formula XIX (280 mg, 0.75 mmol) was stirred in a mixture of acetonitile (6 mL) and trimethylsilylbromide (TMSBr) (574 mg, 3.75 mmol) overnight at room temperature. The solvent was evaporated and the crude product was purified by chromatography on silica gel using dichloromethane:methanol:water (65:35:6) giving 188 mg (91%).
¹H NMR (D₂O) 1.65 (s, 6H), 2.02 (m,2H), 2.42 (s,3H), 3.40 (m, 2H), 4.24 (s, 2H), 5.12 (s, 2H), 8.11 (s,1H).
³¹P NMR (H-decoupled, D₂O): 18.90 (s).

This structure can be represented by formula XX:

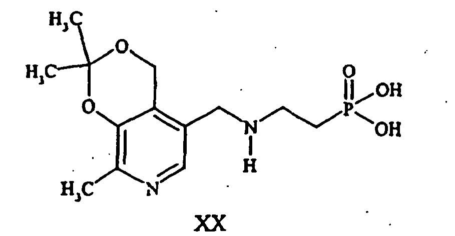

### Example 3: Synthesis of (3-hydroxy-4-hydroxymethyl-2-methyl-5-pyridyl)-3-azabutylphosphonic acid

The product of Example 16, of formula XX (168 mg, 0.53 mmol) was dissolved in acetic acid (80% in water, 10 mL) and heated to 60°C for 5 hours. The solvent was removed by evaporation using toluene to codistill the water. The crude product was purified by chromatography on C-18 reverse phase silica gel using methanol:water (4:1) as eluent to give 57 mg (39%).
¹H NMR (D₂O) 2.05 (m, 2H), 2.52 (s, 3H), 3.38 (m, 2H), 4.42 (s, 2H), 4.96 (s, 2H), 7.87(s, 1H).
³¹P NMR (H-decoupled, D₂O): 18.90 (s).

This structure can be represented by formula XXI:

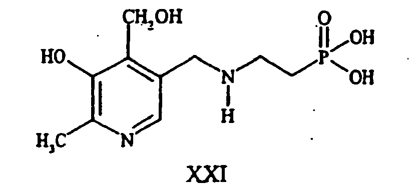

Although embodiments of the invention have been described above, it is not limited thereto, and it will be apparent to persons skilled in the art that numerous modifications and variations form part of the present invention insofar as they do not depart from the scope of the claimed and described invention.

## Claims

1. A compound of the formula II 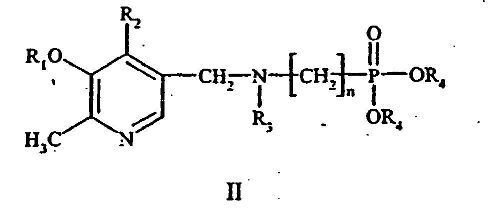 in which
R₁ is hydrogen or alkyl;
R₂ is -CHO, -CH₂OH, -CH₃ or -CO₂R₅ in which R₅ is hydrogen, alkyl, or aryl; or
R₂ is -CH₂₋O-alkyl- (in which alkyl is covalently bonded to the oxygen at the 3-position instead of R₁);
R₃ is hydrogen, alkyl, aryl, or aralkyl;
R₄ is hydrogen, alkyl, aryl, aralkyl, or -CO₂R₆ in which R₆ is hydrogen, alkyl, aryl, or aralkyl; and
n is 1 to 6;
or a pharmaceutically acceptable acid addition salt thereof.

2. The compound of claim 1, wherein
R₁ is hydrogen;
R₂ is -CH₂OH or -CH₂OC(CH₃)₂₋ covalently bonded to the oxgen at the 3-position instead of R₁ to form a six member ring;
R₃ is hydrogen; and
R₄ is hydrogen or ethyl.

3. The compound of claim 1 selected from 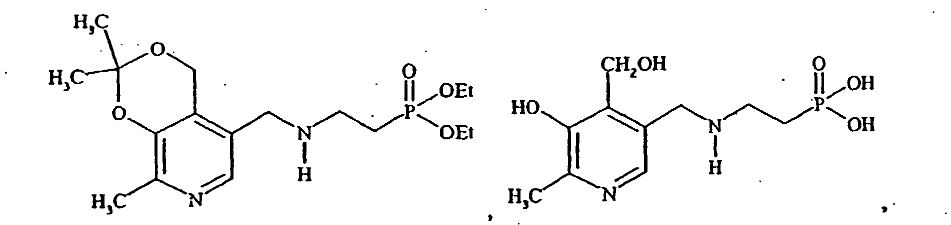 and

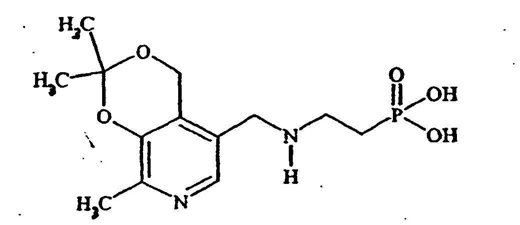

4. A pharmaceutical composition comprising a pharmaceutically acceptable carrier and a therapeutically effective amount of a compound of any of claims 1 to 3.

5. Use of a compound according to any of claims 1 to 3 for the preparation of a medicament for treating cardiovascular said related diseases comprising administering to the mammal a therapeutically effective amount of the compound in a unit dosage form.

6. The use of claim 5, wherein the cardiovascular and related diseases include hypertension, myocardial infarction, ischemia reperfusion injury, myocardial ischemia, congestive heart failure, arrhythmia, blood coagulation, hypertrophy, deep vein thrombosis, disseminated intravascular coagulopathy, or pulmonary embolism.

7. The use of claim 5, wherein the compound of any of claims 1 to 3 is administered concurrently with a therapeutic cardiovascular compound selected from the group consisting of an and angiotensin converting enzyme inhibitor, an angiotensin II receptor antagonist, a calcium channel blacker, an antithrombolytic agent, a β-adrenergic receptor antagonist, a vasodilator, a diuretic, an α-adrenergic receptor antagonist, or a mixture thereof.

8. Use of a compound according to any of claims 1 to 3 for preparation of a medicament for treating diabetes mellitus and related diseases in a mammal comprising administering to the mammal a therapeutically effective amount of the compound in a unit dosage form.

9. The use of claim 8, wherein the diabetes mellitus and related diseases include insulin-dependent diabetes mellitus, noninsulin-dependent diabetes mellitus, insulin resistance, hyperinsulinemia, diabetes-induced hypertension, obesity, or diabetes-related damage to blood vessels, eyes, kidneys, nerves, autonomic nervous system, skin, connective tissue, or immune system.

10. The use of claim 8, wherein the compound of any of claims 1 to 3 is administered concurrently with insulin, a hypoglycemic compound, or a mixture thereof.

11. The use of any of claims 5 to 10, wherein the compound is administered enterally or parenterally.

## Patentansprüche

1. Verbindung der Formel II 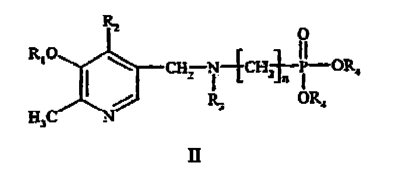 worin
R₁ Wasserstoff oder Alkyl ist;
R₂ -CHO, -CH₂OH, -CH₃ oder -CO₂R₅ ist, worin R₅ Wasserstoff, Alkyl oder Aryl ist; oder
R₂ -CH₂-O-Alkyl- ist (wobei anstelle von R₁ Alkyl kovalent an den Sauerstoff in der 3-Postion gebunden ist);
R₃ Wasserstoff, Alkyl, Aryl oder Aralkyl ist;
R₄ Wasserstoff, Alkyl, Aryl, Aralkyl oder-CO₂R₆ ist, worin R₆ Wasserstoff, Alkyl, Aryl oder Aralkyl ist; und
n 1 bis 6 ist;
oder ein pharmazeutisch annehmbares Säureadditionssalz davon.

2. Verbindung nach Anspruch 1, worin
R₁ Wasserstoff ist;
R₂ -CH₂OH oder -CH₂OC(CH₃)₂- ist, das anstelle von R₁ kovalent an den Sauerstoff in der 3-Position gebunden ist, um einen sechsgliedrigen Ring zu bilden;
R₃ Wasserstoff ist; und
R₄ Wasserstoff oder Ethyl ist.

3. Verbindung nach Anspruch 1, ausgewählt aus 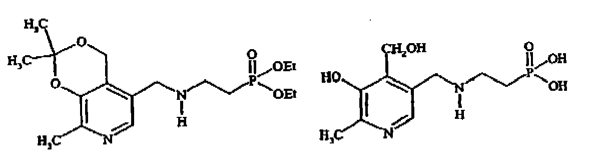 und

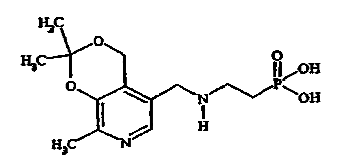

4. Pharmazeutische Zusammensetzung, umfassend einen pharmazeutisch annehmbaren Träger und eine therapeutisch wirksame Menge einer Verbindung nach einem der Ansprüche 1 bis 3.

5. Verwendung einer Verbindung nach einem der Ansprüche 1 bis 3 zur Herstellung eines Medikaments zur Behandlung von kardiovaskulär bedingten Erkrankungen, umfassend das Verabreichen einer therapeutisch wirksamen Menge an Verbindung in einer Einheitsdosisform an das Säugetier.

6. Verwendung nach Anspruch 5, wobei die kardiovaskulären und verwandten Erkrankungen Bluthochdruck, Herzinfarkt, Ischämie-Reperfusionsverletzung, Myocardischämie, kongestives Herzversagen, Arrhythmie, Blutgerinnung, Hypertrophie, Thrombose der tiefen Venen, disseminierte intravaskuläre Koagulopathie oder Lungenembolie umfassen.

7. Verwendung nach Anspruch 5, wobei die Verbindung nach einem der Ansprüche 1 bis 3 gleichzeitig mit einer therapeutischen kardiovaskulären Verbindung verabreicht wird, die aus der Gruppe bestehend aus einem Angiotensin-Converting-Enzymhemmer, einem Angiotensin-II-Rezeptorantagvnisten, einem Kalziumkanalblocker, einem antithrombolytischen Mittel, einem β-adrenergen Rezeptorantagonisten, einem Vasodilatator, einem Diuretikum, einem α-adrenergen Rezeptorantagonisten oder einem Gemisch davon ausgewählt ist.

8. Verwendung einer Verbindung nach einem der Ansprüche 1 bis 3 zur Herstellung eines Medikaments zur Behandlung von Diabetes mellitus und verwandten Erkrankungen bei einem Säugetier, umfassend das Verabreichen einer therapeutisch wirksamen Menge an Verbindung in einer Einheitsdosisform an das Säugetier.

9. Verwendung nach Anspruch 8, wobei der Diabetes mellitus und die verwandten Erkrankungen insulinabhängigen Diabetes mellitus, nichtinsulinabhängigen Diabetes mellitus, Insulinresistenz, Hyperinsulinämie, durch Diabetes hervorgerufenen Bluthochdruck, Fettsucht oder durch Diabetes bedingte Schädigung von Blutgefäßen, Augen, Nieren, Nerven, autonomem Nervensystem, Haut, Bindegewebe oder Immunsystem umfassen.

10. Verwendung nach Anspruch 8, wobei die Verbindung nach einem der Ansprüche 1 bis 3 gleichzeitig mit Insulin, einer hypoglykämischen Verbindung oder einem Gemisch davon verabreicht wird.

11. Verwendung nach einem der Ansprüche 5 bis 10, wobei die Verbindung enteral oder parenteral verabreicht wird.

## Revendications

1. Composé de formule II 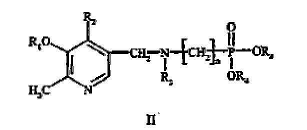 dans laquelle
R₁ est un atome d'hydrogène ou un groupe alkyle;
R₂ est -CHO, -CH₂OH, -CH₃ ou -CO₂R₅, où R₅ est un atome d'hydrogène, un groupe alkyle ou aryle;
ou
R₂ est -CH₂-O-allcyle (où le groupe alkyle est lié par covalence à l'atome d'oxygène en position 3 à la place de R₁) ;
R₃ est un atome d'hydrogène, un groupe alkyle, aryle ou aralkyle ;
R₄ est un atome d'hydrogène, un groupe alkyle, aryle, aralkyle ou -CO₂R₆, où R₆ est atome d'hydrogène, un groupe alkyle, aryle ou aralkyle ; et
n vaut 1 à 6 ;
ou un sel d'addition d'acide pharmaceutiquement acceptable de celui-ci.

2. Composé selon la revendication 1, dans lequel
R₁ est un atome d'hydrogène ;
R₂ est -CH₂OH ou -CH₂OC(CH₃)₂, lié par covalence à l'atome d'oxygène en position 3 à la place de R₁ pour former un cycle à six chaînons ;
R₃ est un atome d'hydrogène ; et
R₄ est un atome d'hydrogène ou un groupe éthyle.

3. Composé selon la revendication 1 choisi parmi 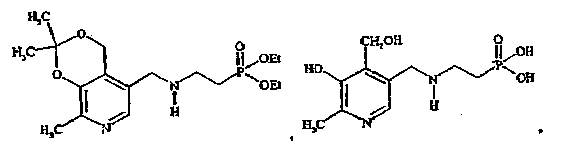 et

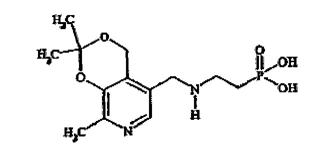

4. Composition pharmaceutique comprenant un véhicule pharmaceutiquement acceptable et une quantité efficace au plan thérapeutique d'un composé selon l'une quelconque des revendications 1 à 3.

5. Utilisation d'un composé selon l'une quelconque des revendications 1 à 3 pour la préparation d'un médicament pour le traitement de maladies cardiovasculaires et apparentées comprenant l'administration à un mammifère d'une quantité efficace au plan thérapeutique du composé sous forme de dose unitaire.

6. Utilisation selon la revendication 5, dans laquelle les maladies cardiovasculaires et apparentées englobent l'hypertension, l'infarctus du myocarde, l'ischémie de reperfusion, l'ischémie du myocarde, l'insuffisance cardiaque congestive, l'arythmie, la coagulation du sang, l'hypertrophie, la thrombose des veines profondes, la coagulopathie intravasculaire disséminée ou l'embolie pulmonaire.

7. Utilisation selon la revendication 5, dans laquelle le composé selon l'une quelconque des revendications 1 à 3 est administré conjointement à un composé cardiovasculaire thérapeutique choisi dans le groupe constitué par un inhibiteur de l'enzyme de conversion de l'angiotensine, un antagoniste du récepteur de l'angiotensine II, un agent bloquant le canal du calcium, un agent antithrombotique, un antagoniste du récepteur β-adrénergique, un vasodilatateur, un diurétique, un antagoniste du récepteur α-adrénergique ou un mélange de ceux-ci.

8. Utilisation d'un composé selon l'une quelconque des revendications 1 à 3 pour la préparation d'un médicament pour le traitement du diabète sucré et des maladies apparentées chez un mammifère comprenant l'administration au mammifère d'une quantité efficace au plan thérapeutique du composé sous forme de dose unitaire.

9. Utilisation selon la revendication 8, dans laquelle le diabète sucré et les maladies apparentées englobent le diabète sucré dépendant de l'insuline, le diabète sucré indépendant de l'insuline, la résistance à l'insuline, l'hyperinsulinémie, l'hypertension induite par le diabète, l'obésité ou une lésion liée au diabète aux vaisseaux sanguins, aux yeux, aux reins, aux nerfs, au système nerveux autonome, à la peau, au tissu connectif ou au système immunitaire.

10. Utilisation selon la revendication 8, dans laquelle le composé selon l'une quelconque des revendications 1 à 3 est administré conjointement à l'insuline, à un composé hypoglycémique ou à un mélange de ceux-ci.

11. Utilisation selon l'une quelconque des revendications 5 à 10, dans lequel le composé est administré par voie entérale ou parentérale.
